# EUROPEAN PATENT SPECIFICATION

(11) **EP 1 719 522 B1**
(45) Date of publication and mention of the grant of the patent: **15.04.2009**
(21) Application number: 06076444.6
(22) Date of filing: 16.07.1993
(51) Int. Cl.: A61K 38/18, A61P 17/02, A61P 41/00, A61P 43/00

(54) **Medicinal composition comprising TCF-II**
TCF-II enthaltende medizinische Zusammensetzung
Composition médicinale comprenant TCF-II

(30) Priority: 16.07.1992 JP 21222792; 16.07.1992 JP 21222992; 10.08.1992 JP 23419892
(43) Date of publication of application: 08.11.2006
(62) Divisional of application: 97100939.4
(73) Proprietor: Daiichi Sankyo Company, Limited, Chuo-ku Tokyo 103-8426 (JP)
(72) Inventor: Fujise, Nobuaki, Hanamakishi Iwate 025-0064 (JP); Higashi, Kanji, Kawagoe-shi Saitama (JP)
(74) Representative: Ellis, Robin Patrick

(56) References cited:
- EP-A- 0 462 277

## Description

### Background of the Invention

This invention relates to medicinal compositions containing an effective amount of TCF-II, more particularly for the healing of injuries caused by incision.

### Description of the Prior Art

Biologically active substances produced by human derived fibroblast cells, for example β-interferon as a tumor cytotoxic factor, have been well known.

Biologically active substances produced by fibroblast cells other than β-interferon such as a tumor cytotoxic glycoprotein called CBF in Japanese Unexamined Patent Publication No. 146293 (1983), a tumor cell proliferation inhibitor (INF) having a molecular weight of 35,000-45,000 in Japanese Unexamined Patent Publication No. 33120 (1986), a tumor proliferation factor (FNF) in Japanese Unexamined Patent Publication No. 1872 (1986), a physiologically active substance having a molecular weight of 40,000-60,000 and an isoelectric point of pH 5.0±0.5 in Japanese Unexamined Patent Publication No. 103021 (1987), and a tumor cytotoxic factor having a molecular weight of 36,000±1,000 and a specific amino acid sequence at isoelectric point of pH 10.5 or higher in Japanese Unexamined Patent Publication No. 10998 (1989), have been known. The inventors have been investigating antitumor substances derived from human fibroblast cells and found a new antitumor proteinous substance. Furthermore, the inventors successfully cloned a cDNA coding for the protein and determined its amino acid sequence. Also the usefulness of the protein was confirmed. The new antitumor protein and its gene were disclosed in the inventors' International Patent Publication No. 10651 (1990). The new antitumor protein was named TCF-II.

TCF-II has both potent anti tumor activity and proliferation stimulative activity for normal cells and is a member of the HGF (hepatocyte growth factor) group. Molecular weight determination of TCF-II with SDS electrophoresis showed 78,000±2,000 or 74,000±2,000. The reduction products of TCF-II showed a common band (A chain) at 52,000±2,000, and two bands. (B and C chains) at 30,000±2,000 and 26,000±2,000, respectively.

Furthermore, therapeutic effect of TCF-II for the healing of injuries caused by incision has not been found.

Skin damage due to wounds and burns has been palliatively treated by external application or oral administration of antibiotics, spreading of acrinol · zinc oxide oil or covering with chitin fibers or lyophilized porcine dermis or by surgical treatments such as sutures and skin grafts.

### Administration of epidermal cell growth factor (EGF) for the regeneration of skin tissue by the proliferation of epithelial cells has been tried but no definite treatment has been established.

### Summary of the Invention

The inventors discovered that TCF-II stimulates the proliferation of epithelial and fibroblast cells to accelerate the healing of injuries caused by incision.

The present invention provides a medicament for the healing of injuries caused by incision comprising TCF-II.

### Detailed Explanation of Preferred Embodiments

The effective ingredient of the present invention is a known glycoprotein (TCF-II) derived from human fibroblast cells as described previously.

TCF-II showed a molecular weight of 78,000±2,000 or 74,000±2,000 in the non-reduced state, and a common band A of 52,000±2,000 and two bands B of 30,000±2,000 and C of 26,000±2,000 in the reduced state by SDS electrophoresis. TCF-II also showed an isoelectric point at pH 7.4-8.6 and was determined as a glycoprotein having a 723 amino acid sequence.

The above mentioned TCF-II can be obtained by evaporation of a human fibroblast cell culture solution, adsorption in an ion exchange resin and affinity chromatography of the eluate (WO90/10651) or by a genetic engineering method (WO92/01053).

TCF-II can be obtained from human fibroblast cells cultured by the method disclosed in WO90/10651. Furthermore, TCF-II produced by a genetic recombination technique using microorganisms or other cells by the gene sequence disclosed in the above mentioned patent publication may be used. The production of TCF-II by the genetic engineering method may be carried out by the method invented by the present inventors and disclosed in WO92/01053. In addition, TCF-II analogues having different sugar chains or no sugar moieties produced by different host cells or microorganisms may also be used. However, presence of sugar moieties is preferable because of their participation in the in vivo metabolic rate.

TCF-II can be concentrated and purified by conventional isolation and purification methods, for example, precipitation with an organic solvent, salting out, gel filtration chromatography, affinity chromatography using a monoclonal antibody and electrophoresis. The purification by affinity chromatography using a monoclonal antibody disclosed in Japanese Patent Application No. 177236 (1991) by the present inventors may be applied.

The resultant purified TCF-II may be kept under lyophilization or deep freezing.

The wound treatment agent of the present invention can be administered around the wound as injection preparations. Direct spreading of TCF-II or homogenous ointments containing TCF-II prepared with fat, fatty oil, lanolin, paraffin, wax, resin, glycols, higher alcohols, glycerin, water, an emulsifier, a suspending agent and so forth may be used. Furthermore, plasters, aerosols, liniments and so forth may be prepared. TCF-II may be adsorbed in sterilized gauze, lyophilized porcine epidermis or chitin fiber used for the protection of wounded skin surface. TCF-II may be administered together with antibiotics, antibacterials and antiseptics, if necessary. Administration to the surgical site of suture may be considered.

The doses of TCF-II included in the wound treatment agents of the present invention can be determined according to the symptoms and conditions of the patients and generally administered at doses of 100-30,000 µg, preferably, 500-3,000 µg of TCF-II to the wound 1-7 times a week. Long term administration may be used according to the symptoms and conditions of the patients.

The present invention will be explained in more detail by the following examples.

### Example 1.

### Purification of TCF-II

Purified TCF-II was obtained by cell culture according to the method disclosed in WO90/10651 or Higashio, K. et al. (B.B.R.C.., 170, 397-404, 1990).

Human fibroblast cells IMR-90 (ATCC CCL 186), 3 x 10⁶ cells, were inoculated in 100 ml of DMEM medium containing 5% bovine serum in a roller bottle and cultured at rotations of 0.5-2/min. for seven days. The culture was continued up to 1 x 10⁷ cells in total, the proliferated cells were separated by treatment with trypsin and collected at the bottom of the bottle. In the bottle, 100 g of sterilized 5-9 mesh ceramic (Toshiba Ceramic Co., Ltd.) was placed and cultured for 24 hrs. upon standing. Then, 500 ml of the culture medium shown above was added to the bottle and cultured further. The total culture medium was recovered every 7-10 days and fresh culture medium was supplied for further culture. Thus, the culture was continued for two months and four 1/bottle of the culture solution was recovered.

The combined culture solution showed specific activity of 32 µ/ml.

Ultrafiltration of 750 l of the cultured solution was performed using a membrane filter (Amicon Corp., MW 6,000 cut) and the filtrate was chromatographed in five steps using CM Sephadex C-50 (Farmacia Biosystems Corp.), ConA Sepharose (Farmacia Biosystems Corp.), MonoS column (Farmacia Biosystems Corp.) and heparin Sepharose (Farmacia Biosystems Corp.) to give purified TCF-II having specific activity of 5,248,000 U/mg.

### Example 2

### Production of gene recombinant TCF-II

TCF-II gene recombinant cells were cultured according to the method disclosed in WO92/01053 and purified TCF-II was obtained. Transformed Namalwa cells were cultured and 20 1 of the culture solution was obtained. The culture solution was treated successively with HPLC using CM-Sephadex C-50 chromato column, Con-A Sepharose CL-6B chromato column and MonoS column to give approximately 11 mg of active TCF-II.

### Example 3

### Production of pharmaceutical compositions of TCF-II

In the present examples, recombinant TCF-II obtained by Example 2 was used for the production of intravenous, subcutaneous and intramuscular injection preparations.

| | | |
|---|---|---|
| (1) | TCF-II | 40 µg |
| | Human serum albumin | 1 mg |

The above composition was dissolved in 0.01M PBS at pH 7.0 and adjusted to 20 ml in total. The solution was sterilized, divided into vials (2 ml each), lyophilized and sealed.

| | | |
|---|---|---|
| (2) | TCF-II | 40 µg |
| | Tween 80 | 1 mg |
| | Human serum albumin | 100 mg |

This composition was dissolved in a saline solution for injection and adjusted to 20 ml in total. The solution was sterilized, divided into vials (2 ml each), lyophilized and sealed.

| | | |
|---|---|---|
| (3) | TCF-II | 20 µg |
| | Tween 80 | 2 mg |
| | Sorbitol | 4 g |

This composition was dissolved in 0.01M PBS at pH 7.0 and adjusted to 20 ml in total. The solution was sterilized, divided intro vials (2 ml each), lyophilized and sealed.

| | | |
|---|---|---|
| (4) | TCFII | 40 µg |
| | Tween 80 | 2 mg |
| | Glycine | 2 g |

This composition was dissolved in a saline solution for injection and adjusted to 20 ml in total. The solution was sterilized, divided into vials (2 ml each), lyophilized and sealed.

| | | |
|---|---|---|
| (5) | TCF-II | 40 µg |
| | Tween 80 | 1 mg |
| | Sorbitol | 2 g |
| | Glycine | 1 g |

This composition was dissolved in a saline solution for injection and adjusted to 20 ml in total. The solution was sterilized, divided into vials (2 ml each), lyophilized and sealed.

| | | |
|---|---|---|
| (6) | TCF-II | 20 µg |
| | Sorbitol | 4 g |
| | Human serum albumin | 50 mg |

This composition was dissolved in 0.01M PBS at pH 7.0 and adjusted to 20 ml in total. The solution was sterilized, divided into vials (2 ml each), lyophilized and sealed.

| | | |
|---|---|---|
| (7) | TCF-II | 40 µg |
| | Glycine | 2 g |
| | Human serum albumin | 50 mg |

This composition was dissolved in a saline solution for injection and adjusted to 20 ml in total. The solution was sterilized, divided into vials (2 ml each), lyophilized and sealed.

| | | |
|---|---|---|
| (8) | TCF-II | 10 mg |
| | Human serum albumin | 100 mg |

This composition was dissolved in 0.01M PBS at pH 7.0 and adjusted to 20 ml in total. The solution was sterilized, divided into vials (2 ml each), lyophilized and sealed.

### Preparation of TCF-II ointment

| | | |
|---|---|---|
| (9) | TCF-II | 1,000 mg |
| | Purified lanolin | 20 g |
| | White soft paraffin | 80 g |

TCF-II was mixed with a small amount of purified water, and mixed and kneaded portionwise with lanolin. White soft paraffin was added portionwise to the resultant mixture and kneaded to give a TCF-II ointment.

| | | |
|---|---|---|
| (10) | TCF-II | 1,000 mg |
| | Macrogol 400 | 5 ml |
| | Macrogol ointment | 100 g |

TCF-II was mixed with a small amount of purified water, and mixed and kneaded portionwise with Macrogol 400. Macrogol ointment was added portionwise to the resultant mixture and kneaded to give a TCF-II ointment.

These pharmaceutical preparations can be used as wound healing agents for healing of injuries caused by incision according to the above mentioned dosage and regimen.

### Experiment 1

### ① Test method

Eight week old male Wistar rats (Charles River Japan, Inc.), 8-9 animals in one group, were sheared on the back and incised approximately 1.5 cm length symmetrically to the median line. Then the incised site was treated with penicillin for the prevention of suppuration and sutured at one point in the center of the incision.

TCF-II was dissolved in pyrogen free PBS containing 0.1% human serum albumin at a rate of one mg/ml and was directly spread on the site of incision immediately after the incision twice a day. The suture was removed on day four, rats were killed on day six and the skin was stripped off. A skin test sample was prepared by cutting the site of incision rectangularly. The obtained test sample was pulled at both ends to determine its tensile force until the sutured site was broken. The determined value with FD pickup (Nihon Kohden Corp., TB-611T) was recorded in a polygraph (Nihon Kohden Corp., RM-6200) via an amplifier for strain pressure (Nihon Kohden Corp., AP-601G). The control group was prepared by spreading solely the solvent, 0.1% human serum albumin containing PBS, instead of the test solution.

### ② Test results

The maximum tension for the break of the suture is shown in Table 4.

**Table 4 Tensile force of rat skin at the site of suture**

| Treatment | Tensile force (g) (Mean ± S.E.) |
|---|---|
| Control (n = 9) | 190.06±26.2 |
| TCF-II (n = 8) | 314.4 ± 24.4** |

| | |
|---|---|
| ** P<0.01 (Wilcoxon test) | |

As shown in the Table 4, the bond strength at the site of incision in TCF-II administered group was approximately 1.65 times that of the control group indicating a wound healing effect of TCF-II.

## Claims

1. Use of TCF-II in the preparation of a medicament for the healing of injuries caused by incision.

2. Use according to claim 1 in which the medicament prepared is In injectable form or ointment.

3. A pharmaceutical composition comprising TCF-II for use in the healing of injuries caused by incision.

4. A pharmaceutical composition according to claim 3 which is in injectable form or is in the form of an ointment.

## Patentansprüche

1. Verwendung von TCF-II bei der Herstellung eines Medikaments zum Heilen von durch Schnitt verursachten Verletzungen.

2. Verwendung nach Anspruch 1, wobei das hergestellte Medikament in injizierbarer Form oder als Salbe vorliegt.

3. Pharmazeutische Zusammensetzung, die TCF-II zur Verwendung beim Heilen von durch Schnitt verursachten Verletzungen enthält.

4. Pharmazeutische Zusammensetzung nach Anspruch 3, die in injizierbarer Form oder in Form einer Salbe vorliegt.

## Revendications

1. Utilisation de TCF-II dans la préparation d'un médicament pour la cicatrisation de plaies causées par une incision.

2. Utilisation selon la revendication 1, dans laquelle le médicament préparé est sous forme injectable ou de pommade.

3. Composition pharmaceutique comprenant du TCF-II à utiliser pour la cicatrisation de plaies causées par une incision.

4. Composition pharmaceutique selon la revendication 3, qui est sous forme injectable ou qui est sous forme de pommade.
